# EUROPEAN PATENT APPLICATION

(11) **EP 3 757 230 A1**
(43) Date of publication of application: **30.12.2020**
(21) Application number: 19757401.5
(22) Date of filing: 20.02.2019
(51) Int. Cl.: C12Q 1/6876, C12Q 1/686, C12R 1/21, C12R 1/36, C12R 1/46

(54) **OLIGONUCLEOTIDE, SET OF OLIGONUCLEOTIDES, METHOD FOR SIMULTANEOUS DETECTION OF NEISSERIA MENINGITIDIS, STREPTOCOCCUS PNEUMONIAE AND HAEMOPHILUS INFLUENZAE, AND KIT**

(30) Priority: 20.02.2018 BR 102018003245
(71) Applicant: Fundação Oswaldo Cruz, 21045-900 Rio de Janeiro, RJ (BR)
(72) Inventor: CAPASSO, Ivano Rafaelle Victorio de Filippis, 22061- 040 Rio de Janeiro, RJ (BR); DE ALMEIDA, Antonio Eugenio Castro Cardoso, 22230- 001 Rio de Janeiro, RJ (BR)
(74) Representative: ZBM Patents - Zea, Barlocci & Markvardsen
(86) International application number: PCT/BR2019/050049
(87) International publication number: WO 2019/161469

(57) **Abstract**

The present invention provides a real-time PCR method that allows in a single step the simultaneous detection of etiological agents of bacterial meningitis, more specifically, *Neisseria meningitidis, Streptococcus pneumoniae* and *Haemophilus influenzae.* For this, primers were used to amplify particular regions of the genomes of said bacteria. The presence of bacteria in a sample is indicated by the presence of amplicon, which is detected by means of detection methods appropriate to the PCR methodology employed.

## Description

### Field of the invention

The invention relates generally to the amplification and detection of nucleic acids. In particular, methods, oligonucleotides and a diagnostic *kit* are provided to simultaneously confirm and discriminate, in a single step, the etiological agents of bacterial meningitis, more specifically, *Neisseria meningitidis, Streptococcus pneumoniae* and *Haemophilus influenzae.*

### Background of the Invention

Bacterial meningitis is a worldwide public health problem. Meningitis is a serious infection and causes inflammation in the membranes lining the brain affecting the central nervous system. The three main etiological agents of bacterial meningitis are *Neisseria meningitidis, Haemophilus influenzae* and *Streptococcus pneumoniae,* representing more than 80% of cases of bacterial meningitis. In addition to meningitis, these agents can cause invasive disease by invading the bloodstream, which may or may not be associated with meningitis. In cases of suspected meningitis, blood or CSF samples <are collected and analyzed by microbiological or immunological laboratory methods. The rapid detection of the etiologic agent facilitates the patient's treatment choice, leading to a better prognosis. Prophylactic antibiotics are administered to the patient's contacts to reduce the transmission and spread of the infection. Conventional laboratory tests can take more than 36 hours to diagnose from suspicious clinical material and a large number of false negative results can be released due to the fastidious nature of the etiological agents, making their detection difficult by conventional methods. For these reasons, the rapid diagnosis <of these infections is essential to determine the appropriate treatment, avoiding serious consequences for the patient and helping epidemiological monitoring.

The three main etiological agents of bacterial meningitis *(Neisseria meningitidis, Streptococcus pneumoniae* and *Haemophilus influenzae)* are characterized as bacteria that are difficult to cultivate and detect in clinical material, by conventional laboratory methods. The injuries caused by these pathogens are considered to be very serious with rapid evolution and 20% lethality or sequelae such as deafness, brain damage and amputation of limbs or extremities. For these reasons, the rapid diagnosis of these agents is extremely important for the rapid management of patients, enabling a better prognosis. Some molecular diagnostic methods have already been developed for the detection of these pathogens. These methods are based on the polymerase chain reaction (PCR) and can be in the conventional way or by conventional PCR (qPCR), which is more sensitive, with the Taqman system being the most used. The Taqman system uses the same reagents as the conventional PCR, with the addition of a fluorescent probe to indicate the presence of the target and, consequently, the positive diagnosis. These probes have a very high cost compared to the other reagents used in the reaction and because they are three different targets, it is necessary to use three different probes, making the system even more expensive.

Therefore, there is a need in the art for a method of rapid and economically effective diagnosis, which allows the detection and discrimination of the aforementioned etiological agents.

The present inventors have developed a more economical and faster method, based on real-time PCR.

The present invention therefore consists of designing primers for detecting and discriminating the etiological agents of bacterial meningitis *Neisseria meningitidis, Streptococcus pneumoniae* and *Haemophilus influenzae* by real-time PCR (qPCR). These primers and probes form a protocol for simultaneous detection of the aforementioned etiologic agents, using the qPCR methodology.

The main benefits of the technology are: the possibility of a quick identification of the etiological agents of bacterial meningitis, by real-time PCR, in addition to the best prognosis for patients. In addition, greater scope is also provided for the diagnostic examination of bacterial meningitis, as it has a potentially lower cost than that currently used. It is worth mentioning that despite the drastic cost reduction provided by the methods and kits of the present invention, the quality of the analysis is maintained with high sensitivity and specificity.

Thus, the implementation of the technology described here may mean meeting the need for methodologies for the safe conclusion of the diagnosis, with high specificity and sensitivity.

The invention will be presented in more detail below.

### Summary of the Invention

In one aspect, the invention provides oligonucleotides capable of detecting the DNA of the etiologic agents of bacterial meningitis differentially.

In a particular aspect, the invention provides primers for use in the method of the present invention.

In a particular aspect, the invention provides probes for use in the method of the present invention.

In a particular aspect, the primers used in the detection of *N. meningitidis* DNA target the nspA *gene.* In a more specific aspect, the primers for detecting the *nspA* gene are shown in SEQ ID NOs: 1 and 2.

In a particular aspect, the primers used in the detection of *H. influenza* DNA target the *P6* gene. In a more specific aspect, the primers for detecting the *P6* gene are shown in SEQ ID NOs: 3 and 4.

In a particular aspect, the primers used in the detection of *S*. *pneumoniae* DNA target the *ply* gene. In a more specific aspect, the primers for detecting the *ply* gene are shown in SEQ ID NOs: 5 and 6.

In one embodiment, the oligonucleotide suitable as a probe is labeled with a detectable label, preferably a fluorescent cluster, comprising a donor fluorophore pair and a *quencher.*

In another particular aspect, the probe for the detection of *N. meningitidis* has the sequence shown in SEQ ID NO: 7.

In another particular aspect, the probe for detecting *H*. *influenzae* has the sequence shown in SEQ ID NO: 8.

In another particular aspect, the probe for the detection of *S*. *pneumoniae* has the sequence shown in SEQ ID NO: 9.

In another additional aspect, a method is provided for the detection and discrimination of the etiological agents of bacterial meningitis, comprising the steps of:
a) producing at least one amplicon using at least two primers oligonucleotides, such oligonucleotides as defined above;
b) and detecting at least one amplicon.

In an optional embodiment, the amplicon is detected by at least one oligonucleotide probe.

In an optional embodiment, amplicon is detected through *melting temperature differences (TM).*

In a particular embodiment, the amplicon of *N. meningitidis* has a TM=85.8°C. In another embodiment, the *H. influenzae* amplicon has a TM =80°C. In another modality, the *S. pneumoniae* amplicon has a TM=77°C.

In one embodiment of aforementioned method, the referred step of producing at least one amplicon comprises at least one of amplification by *uniplex, multiplex,* qualitative, or real-time PCR.

In another embodiment, the method makes it possible to discriminate between infections by *N. meningitidis, H. influenzae* and *S*. *pneumoniae.*

Another aspect of the invention concerns a kit for diagnosing and discriminating against infection by *N. meningitidis, H. influenzae* and *S*. *pneumoniae,* comprising: a) at least one pair of oligonucleotides suitable as a primer; and/or b) optionally at least one oligonucleotide suitable as a probe; and c) optionally, instructions for use.

In one embodiment, the *kit* of the invention further includes oligonucleotide primers capable of amplifying and discriminating the etiological agents of bacterial meningitis. In a further embodiment, these bacterial etiologic agents are selected from *N. meningitidis, H. influenzae* and *S. pneumoniae.*

In an optional embodiment, the kit includes probe oligonucleotides that allow the detection of the amplicon obtained from the amplification using the oligonucleotide primers of the present invention.

Additionally, in one embodiment, the *kit* of the invention still includes a negative control and/or a positive reaction control.

### Brief Description of the Images

**Image 1**. PCR by the Taqman uniplex system with *N*. *meningitidis* to determine the detection limit (LD). 1A: 242 ng/µL and 242 pg/µL. **1B:** 2.42pg/µL and 242 fg/µL.
**Image 2.** PCR by the Taqman uniplex system with *H*. *influenzae* to determine the LD. 2A 109 ng/µL and 2B: 1.09 pg/µL and 109 fg/µL
**Image 3.** PCR using the Taqman uniplex system with *S*. *pneumoniae* to determine the LD. 3A: 0.479 ng/µL. 3B: 479 fg/µL and 200 fg/µL
**Image 4.** PCR by the Taqman multiplex system with *N*. *meningitidis* to determine the LD with 2.42 ng/µL, 242 pg/µL and 242 fg/µL.
**Image 5.** PCR by the Taqman multiplex system with *H*. *influenzae* to determine the LD with 1.09 ng/µL, 1.09 pg/µL and 200 fg/µL.
**Image 6.** PCR by the Taqman multiplex system with *S*. *pneumoniae* to determine the LD with 0.479 ng/µL, 4.79 pg/µL and 200 fg/µL.
**Image 7.** PCR by the uniplex HRM system with *N*. *meningitidis* to determine the LD with 2.42 ng/µL, 2.42 pg/µL and 242 fg/µL.
**Image 8.** PCR by the HRM system uniplex with *H. influenzae* to determine the LD with 1.09ng/µL, 1.09pg/µL and 200fg/µL.
**Image 9.** PCR by the uniplex HRM system with *S*. *pneumoniae* to determine the LD with 0.47 ng/µL, 479 fg/µL and 200 fg/µL.
**Image 10.** PCR by the uniplex/multiplex HRM system of *S*. *pneumoniae* INCQS 00543 (BinC), *H influenzae* INCQS 00434 (BinB) e *N. meningitidis* INCQS 00140 (BinA), showing the standard dissociation curves (TM) for the three targets. The detection of each agent is directly associated with the respective temperatures of 77°C, 80°C and 85.8°C.
**Image 11.** PCR by the Taqman multiplex system with detection of reference *N meningitidis* (INCQS 00140) from clinical material, indicating the detection of the etiological agent by the correlation with the specificity of the oligos and the wavelength of the fluorophore channel (FAM).
**Image 12.** PCR by the Taqman multiplex system with detection of reference S. *pneumoniae* (INCQS 00543) from clinical material, indicating the detection of the etiological agent by the correlation with the specificity of the oligos and the wavelength of the fluorophore channel (Cy5).
**Image 13.** PCR by the HRM multiplex system with detection of reference S. pneumoniae (INCQS 00543) and from clinical material, indicating the detection of the etiological agent by the correlation with the *melting* temperature (TM=77°C).
**Image 14.** PCR by the HRM multiplex system with detection of reference *N. meningitidis* (INCQS 00164) and from clinical material, indicating the detection of the etiologic agent by the correlation with the melting *temperature* (TM=85.8°C).
**Image 15.** PCR by the HRM multiplex system with negative controls according to Table 1 (below). The oligonucleotides showed specificity for the target microorganisms, with no interference when negative controls were used.

### Detailed Description of the Invention

Unless defined differently, all technical and scientific terms used herein have the same meaning as understood by an experienced technician to which the invention belongs. Conventional molecular biology techniques are well known to an experienced technician, and can be found, for example, in Ausubel et al., eds. Current Protocols in Molecular Biology, John Wiley & Sons, Inc. NY (1987-2008), including all supplements; Sambrook et al., Molecular Cloning: A Laboratory Manual, 2nd edition, Cold Spring Harbor, N.Y. (1989). The specification also provides definitions of terms to assist in the interpretation of what is described here and the claims.

Unless otherwise indicated, all numbers expressing quantities, percentages and proportions, and other numerical values used in the specification and in the claims, should be understood as being modified, in all cases, by the term "approximately". Thus, unless otherwise indicated, the numerical parameters shown in the specification and in the claims are approximations that may vary, depending on the properties to be obtained.

The invention described here relates to new oligonucleotides for amplification, detection, differentiation of the etiological agents of bacterial meningitis, particularly, *N. meningitidis, H. influenzae* and *S*. *pneumoniae* and related methods and *kits.* More specifically, the present invention provides oligonucleotides, including primers and, optionally, probes, which are suitable for the detection and discrimination of *N. meningitidis, H. influenzae* and *S. pneumoniae.*

### Oligonucleotides and diagnostic kits

"Oligonucleotide" refers to any short polymer of nucleotides, wherein the nucleotides can be ribonucleotides, deoxyribonucleotides, dideoxyribonucleotides, degenerate nucleotides, and similar. Such oligonucleotides are preferably single-stranded. The length of such oligonucleotides may vary, and is usually less than 150 nucleotides (nt), preferably in the range of 10-100 nt, more preferably in the range of 10-60 nt, even more preferably of 13-50 nt. They may also have chemical modifications, such as a label or a tag, for example, fluorescent, radioactive, biotinylated, DIG (digoxigenin), and similar. The oligonucleotides of the invention can be either *forward* (sense) or reverse (antisense).

In one aspect, the oligonucleotides according to the present invention include primers and, optionally, probes. Unless otherwise stated, strings are displayed in the 5' to 3' direction. Such oligonucleotides can be in various forms, for example, in solution/suspension in a suitable solvent and in a desired concentration, dried or lyophilized. The technician on the matter is aware of the solvents, concentrations and storage conditions suitable for the oligonucleotides of the invention. In particular, the experienced technician is aware of how to prepare such oligonucleotides as stock solutions. The oligonucleotides according to the invention can have varying degrees of purity, which can be evaluated by an experienced technician, for example, using HPLC chromatography.

In addition, it should be remembered that, although the preferred functions may be mentioned in relation to some oligonucleotides, it is obvious that a given oligonucleotide can take on several functions, and can be used in different forms according to the present invention. As is known to the technician on the matter, in some situations, the sequence of a primer can be used as a probe and vice versa, in addition to being applicable in hybridization procedures, detection etc. Thus, it is observed that the products according to the present invention, especially, *inter alia,* oligonucleotides, are not limited to the uses shown here, but, on the contrary, the uses must be interpreted broadly, regardless of the use indicated here.

In addition, when an oligonucleotide is described as being useful as a probe capable of binding to an amplicon, the technician on the matter also understands that the complementary sequence of this oligonucleotide is also useful as a probe for binding to the same amplicon. The same is true for sequences described as useful as primers. In addition, it is also obvious that any suitable primer for a *multiplex* protocol can also, within the meaning and scope of the present invention, be used in a *singleplex protocol.* The same applies to a suitable primer for a real-time PCR protocol, which can be used in a conventional PCR protocol, within the meaning of the present invention.

The terms "hybridize" and "annular" are used interchangeably and mean the base-pairing interaction of a nucleic acid with another nucleic acid that results in the formation of a double, triple, or other more complex strand. In some embodiments, the primary interaction is specific, for example, C/G and A/T, by hydrogen bonding.

The technician on the matter, in this regard, understands that the oligonucleotides of the present invention, i.e., primers and probes, need not be completely complementary to a part of the target sequence. The primer can be sufficiently complementary to hybridize to the target sequence and perform the intrinsic functions of a primer. The same applies to a probe, that is, a probe can be sufficiently complementary to hybridize with the target sequence and perform the intrinsic functions of a probe. Therefore, a primer or probe, in one embodiment, does not need to be completely complementary to the target sequence. In one embodiment, the primer or probe can hybridize or anneal to a portion of the target to form a double strand. Hybridization conditions for a nucleic acid are described by Joseph Sambrook et al., Molecular Cloning, A Laboratory Manual, Cold Spring Harbor Laboratory Press, Cold Spring Harbor, N.Y. (2001) and Haymes et al., Nucleic Acid Hybridization, A Practical Approach, IRL Press, Washington, D.C. (1985). Thus, since there is no need for complete complementarity to occur annealing, a technician on the matter will understand that the sequences of primers and probes described here can be modified to some degree without losing their usefulness as specific primers and probes for *Neisseria meningitidis, Haemophilus influenzae* and *Streptococcus pneumoniae.*

Regarding the definition of "primer", a technician on the matter knows that it includes any single-stranded oligonucleotide capable of annealing to a complementary target mold, under conditions of adequate stringency, and that serves as a starting point for the synthesis of a product of extension (amplicon) from the primer, by elongating the strand by a DNA polymerase under suitable conditions. These conditions include 4 different types of deoxynucleoside triphosphates and DNA polymerase or reverse transcriptase under suitable temperature conditions and in a suitable buffer solution. The length of the primer can vary according to several factors, but the typical length of a primer is 10-50 nt, preferably 15-30 nt, more preferably 15, 16, 17, 18, 19, 20, 21, 22, 23, 24, 25, 26, 27, 28, 29 or 30 nt. According to the present invention, it is preferable for each primer to be 15-30 nt. The *forward* and *reverse* primers are primers that bind, respectively, to a 3' end and a 5' end of a specific region of the target that is amplified by the PCR reaction.

The specific oligonucleotides for each species for use in the Taqman and HRM system of real-time PCR, were designed from the sequences of the target genes unique to each microorganism with the aid of the Oligo Architect SIGMA software (http://www.oligoarchitect.com/). The strings were obtained from GenBank (http://www.ncbi.nlm.nih.gov/genbank/).

Preferably, these primers are, but are not particularly limited to, a primer comprising at least 10 to 15 consecutive nucleotides of any of the sequences described in SEQ ID Nos: 1 to 6, and their complementary sequences. The primers of SEQ ID Nos: 1 and 2 are capable of amplifying a region of the *N. meningitidis* nspA gene; whereas the primers of SEQ ID Nos: 3 and 4 are capable of amplifying a region of the *H. influenzae* P6 gene; while the primers of SEQ ID Nos: 5 and 6 are capable of amplifying a region of the *S. pneumoniae* ply gene. The primer can also consist of any fragment of the sequences of SEQ ID Nos: 1 to 6 capable of amplifying the target genes of the species of *N. meningitidis, H. influenzae* and *S. pneumoniae,* and their complementary sequences.

In an alternative embodiment, a probe is used to detect the amplicon obtained with the PCR. The probe definition is also known to a technician on the matter, and includes any oligonucleotide that is capable of hybridizing to a complementary target sequence under suitable hybridization conditions. Once the probe is labeled, it can be used to detect the presence of certain nucleotide sequences. The probes can be prepared in the form of single stranded DNA, double stranded DNA, RNA or hybrid DNA-RNA. The typical length of a probe is 10-60 nt, preferably 15-55 nt, more preferably 20-50 nt, more preferably 30-45 nt, even more preferably 10-30 nt. Thus, according to the present invention, the probe can include or comprise at least 8-15 consecutive nucleotides from the sequences described in SEQ ID Nos: 7 to 9. The probe can also comprise or consist of any of the sequences of SEQ ID Nos: 7 and 9, and their complementary sequences.

Each probe was marked with a different fluorophore so that they could be used simultaneously in a multiplex system. The appropriate quencher was used for *each* fluorophore.

Various probe formats can be used to perform real-time PCR, such as fluorescently labeled probes. More specifically, the probes can be of the FRET (*fluorescence resonance energy transfer*) type which include, but are not limited to, TaqMan™, Molecular Beacon™, Scorpion™, and LUX™ probes. In a preferred embodiment, the probes according to the invention are of the TaqMan™ type.

More specifically, regarding the TaqMan™ probe, an oligonucleotide, whose 5' terminal region is modified with a fluorophore and the 3' terminal region is modified with a *quencher,* is added to the PCR reaction. It is also understood that it is possible to bind the fluorophore in the 3' terminal region and the *quencher* in the 5' terminal region. The reaction products are detected by the fluorescence generated after the 5' -> 3' exonuclease activity of DNA polymerase. Fluorophores, which refer to fluorescent compounds that emit light with excitation by light having a shorter wavelength than the light that is emitted, can be, but are not limited to, FAM, TAMRA, VIC, JOE, TET, HEX, ROX, RED610, RED670, NED, Cy3, Cy5, and Texas Red. *Quenchers* can be, but are not limited to, 6-TAMRA, BHQ-1,2,3 and MGB-NFQ. The choice of the fluorophore-*quencher* pair can be made so that the excitation spectrum of the *quencher* has an overlap with the emission spectrum of the fluorophore. An example is the pair FAM-TAMRA, FAM-MGB, VIC-MGB and so on. A technician on the matter will know how to recognize other suitable pairs.

In a preferred embodiment according to the invention, the spectrum properties of such probes are chosen so that one probe does not interfere with the other. In particular, when the probes are used in *multiplex* reactions, each probe will have its own fluorophore being spectral and significantly different from another probe, that is, the absorption/emission spectra of the different probes are essentially non-overlapping. This advantageously allows the detection of each probe individually, since the individual signals do not interfere with each other during detection.

The fluorescence emitted during the target nucleic acid amplification reaction is measured in order to monitor the accumulation of specific amplification products. The fluorescence signal is proportional to the amount of the specific amplicon produced. In the presence of the target sequences of *N. meningitidis, H. influenzae* and *S. pneumoniae,* fluorescence will increase. In the absence of the target sequences, fluorescence will remain consistently low throughout the reaction.

In a preferred embodiment, fluorophores are selected from the group consisting of HEX, Cys-5 and FAM.

In a preferred embodiment, *quenchers* are selected from the group consisting of BHQ-1 and 3.

In addition, in one embodiment, to provide a standard (internal control) for determining nucleic acid extraction from a biological sample to be tested, potentially comprising the target sequence of *N. meningitidis, H. influenzae* and *S. pneumoniae,* or to determine the presence or absence of potential reaction inhibitors, primers capable of amplifying, and probes capable of detecting, amplicons resulting from the amplification of human endogenous sequences can be used. A non-limiting example is to use primers that target, for example, the RNAs of human beta-globin, beta-actin, RNase and GAPDH.

Also, to provide a positive control for the amplification of the targets and/or to facilitate the quantification of the etiological agents of interest in a given biological sample to be analyzed, an external positive control can be incorporated. In the present invention, reference strains from different serogroups and serotypes can be used, a nucleic acid sample containing copies of the targets of *N. meningitidis, H. influenzae* and *S. pneumoniae,* for example, a cassette or vector comprising the target sequence to be amplified. Illustratively, and in a non-limiting way, the reference serotypes and serogroups used by the present inventors are listed in Table 1, below, where the reference microorganisms to be detected are also listed.

Similarly, a negative reaction control can be incorporated. This control can be a nucleic acid sample that does not contain any copies of the target gene of *N. meningitidis, H. influenzae* and *S. pneumoniae,* for example, samples taken from reference bacterial species other than *N. meningitidis, H. influenzae* and *S. pneumoniae.* Illustratively, but not limited to, the present inventors used as reference microorganisms for negative control *Neisseria perflva* (ATCC11076), *Moraxella catarralis* (ATCC25238), *Streptococcus agalactiae* (ATCC 13813), *Streptococcus pyogenes* (ATCC 19615), *Klebsiella pneumoniae* (ATCC 13883), *Listeria monocytogenes* (ATCC 15313), *Acinetobactersp.* (ATCC 14293) or *Escherichia coli* (ATCC 11775).

Another aspect of the invention is a *kit* used to diagnose and differentiate infections caused by *N. meningitidis, H. influenzae* and *S. pneumoniae,* simultaneously, comprising at least one set of oligonucleotides. By "oligonucleotide set" is meant any combination comprising at least one oligonucleotide, preferably at least two, for example, from 2 to 20 oligonucleotides. Such set can, for example, comprise at least one primer, or at least a pair of primers. Alternatively, such set may comprise, in addition to at least one primer or at least a pair of primers, at least one probe. Such oligonucleotides can be kept separate, or partially mixed, or completely mixed.

Preferably, such *kit* comprises at least one set of oligonucleotides according to the invention, designed specifically for *N. meningitidis, H. influenzae* and *S. pneumoniae.* Such oligonucleotides can be maintained either separately, or partially mixed, or fully mixed. Oligonucleotides can be provided in dry form, or solubilized in a suitable solvent, according to the knowledge of the art. For example, suitable solvents include TE, ultrapure water, and similar.

In one embodiment, the *kit* according to the invention may also contain additional reagents suitable for the amplification reaction, including water, nuclease-free water, RNase-free water, DNase-free water, ultrapure water, salts (such as magnesium, potassium salts), buffers (such as conventional PCR buffers, known in the art), enzymes, including thermostable polymerases, such as Taq, Vent, Pwo, Pfu, reverse transcriptase, and the like, nucleotides such as deoxynucleotides, dideoxynucleotides, dNTPs, dATP, dTTP, dCTP, dGTP, dUTP, other reagents, such as additives, RNase or DNase inhibitors, and polynucleotides such as poliT, polidT, and other oligonucleotides, as primers and probes for other pathogens, and for internal controls, as a control positive (e.g. human beta-globin) or a phage (e.g. MS2). The reagents can be provided in a concentrated form for dilution to an appropriate concentration by the end user. In addition, at least part of the reagents can be provided as a pre-mix.

Such reagents can be accommodated in containers, which for the purposes of the present invention include, but are not limited to, microtubes, tubes, PCR plates with different amounts of wells, chips, or any other suitable and inert medium where the amplification reaction occur, and does not react with the fluids and solutions of the present invention. In addition, the container can also be labeled and identified, for example, with colors, to avoid confusion and provide ease of use for a technician in the laboratory.

Beyond that, in one embodiment, the *kit* according to the invention contains instructions for its use. These instructions can be on a brochure, card, or similar. These instructions can be in two forms: detailed, providing exhaustive information regarding the *kit* and its use, possibly also including literature data; and a simple one, in the form of a quick guide, bringing essential information needed to use the *kit.*

In a preferred form, such *kit* is a diagnostic *kit,* especially an *in vitro* diagnostic *kit.* More preferably, such *kit* is a *kit* for the diagnosis and differentiation of *N. meningitidis, H. influenzae* and *S. pneumoniae.*

In another embodiment of the present invention, the diagnostic *kit* can further include a *kit* for extracting and isolating nucleic acids from a biological sample. Such extraction *kit* may comprise a lysis buffer, a wash buffer and an elution buffer. The extraction *kit* can also be provided with empty containers and adsorption columns for extraction and isolation of nucleic acids.

Polynucleotides of etiological agents of meningitis, more specifically, *N. meningitidis, H. influenzae* and *S. pneumoniae,* are the targets or source of the target for the amplification reaction of the present invention. The term "target sequence", or simply "target", refers to a nucleic acid sequence that serves as a template for amplification in a PCR reaction. These nucleic acid sequences can contain deoxyribonucleotides, ribonucleotides, and/or their analogs. The sequence can be a gene or gene fragment, mRNA, cDNA, isolated total DNA, isolated total RNA, and similar.

More specifically, the target sequences of the present invention are transcribed *from* the *N. meningitidis* nspA gene; *H. influenzae* P6 gene; and *S. pneumoniae ply gene.*

In one embodiment, the target sequence is present in a sample of biological material collected from an individual. By "sample", therefore, is meant any biological substance or material that may contain some etiological agent of bacterial meningitis, particularly, one or more strains of *N. meningitidis, H. influenzae* or *S. pneumoniae.* The samples include, but are not limited to, blood, plasma, serum, liquor and similar.

Procedures for the extraction and purification of nucleic acids are well known in the art. Examples of methods for extracting nucleic acids from whole blood are taught, for example, in Casareale et al. (Genome Res., 2: 149-153, 1992) and in U.S. Patent No. 5,334,499.

Once primers are prepared, target nucleic acid amplification can be performed by a variety of methods, including, but not limited to, conventional PCR, real-time PCR, RT-PCR, nested-PCR, semi-PCR quantitative and others. Preferably, the method used is real-time PCR.

"Amplification" refers to nucleic acid amplification procedures using primers and polymerases that generate multiple copies of a target nucleic acid. Such amplification reactions are known to the experienced technician as "PCR" (polymerase chain reaction), which in turn includes, for the purposes of this invention, any method based on PCR, including conventional, qualitative, semi-quantitative PCR, real-time, reverse transcription reaction (RT-PCR), *singleplex* PCR, *multiplex,* and similar.

An "amplicon" or "PCR product", the terms being used interchangeably, refer to a nucleic acid (or collectively, the plurality of nucleic acid molecules) that was synthesized during the amplification procedures. An amplicon is typically, but not exclusively, a DNA fragment.

By "reverse transcription" (RT) is meant the phenomenon in which a copy of cDNA is produced from an RNA molecule. The resulting cDNA can be used as a template for PCR.

"RT-PCR" means a reaction in which an RNA molecule is reverse transcribed to produce a product (cDNA) and the cDNA is subsequently amplified in a PCR reaction. Typically, both reverse transcription and the PCR reactions of the RT-PCR reaction are performed in a single tube.

By "quantitative PCR" (qPCR), is meant any method based on PCR that allows the estimation of the initial quantity of a given target sequence in a given sample.

"Real-time PCR" means any PCR-based method that allows monitoring the fluorescence emitted during the reaction, as an indicator of the production of the PCR product or amplicon during each PCR cycle, as opposed to detection at the end of the run of all cycles in conventional PCR methods.

As used here, *"multiplex* PCR" refers to any PCR reaction that aims to simultaneously amplify more than one target. For example, *multiplex* PCR includes *biplex* PCR (two targets), triplex PCR (three targets), and so on. *Multiplex* PCR includes PCR reactions with more than one pair of primers, for example, two pairs of primers. In this case, there may be four different primers, but it is also possible that there is a common primer, for example, the *forward* primer, and two different reverse primers. *Multiplex* PCR also includes PCR reactions with a single pair of primers, but with more than one probe. Also, as non-limiting examples, *multiplex* amplification includes amplification reactions from different genes, different alleles from a single gene and/or different fragments from a single gene.

A "buffer" is a composition added to the amplification reaction, comprising a buffering agent, which modifies the stability, activity and/or longevity of one or more components of the amplification reaction, by regulating the pH of the amplification reaction. The buffering agents of the invention are compatible with the activity of the polymerase to be used, that is, a DNA polymerase. Buffering agents are well known in the art and include, but are not limited to Tris, Tricin, MOPS (3- (N-morpholino) propane-sulfonic acid), and HEPES (4- (2-hydroxyethyl) -1-piperazine acid-ethanesulfonic).

In addition, PCR buffers can generally contain up to about 70 mM KCl and about 1.5 mM or more MgC12, at about 50-500 µM of each of the dATP, dCTP, dGTP and dTTP nucleotides.

The buffers of the invention may also contain additives. An additive is a compound added to a composition that modifies the stability, activity and/or longevity of one or more components of the composition. In some embodiments, the composition is an amplification composition. In some embodiments, an additive inactivates contaminating enzymes, stabilizes protein folding and/or decreases aggregation. According to the invention, additives can be added to improve the selectivity of the annealing of a primer and/or a probe, as long as the additive does not interfere with DNA polymerase activity.

Examples of additives are, but are not limited to, betaine, glycerol, formamide, KCl, CaC12, MgOAc, MgC12, NaCl, NH4OAc, NaI, Na (CO3) 2, LiCl, MnOAc, NMP, trehalose, DMSO, ethylene glycol, dithiothreitol ("DTT"), pyrophosphatase (including, but not limited to, inorganic *Thermoplasma acidophilum* pyrophosphatase ("TAP")), bovine serum albumin ("BSA"), propylene glycol, glycinamide, CHES, Percoll ™, aurintricarboxylic acid, Tween 20, Tween 21, Tween 40, Tween 60, Tween 85, Brij 30, NP-40, Triton X-100, CHAPS, CHAPSO, Mackernium, LDAO (N-dodecyl-N, N-dimethylamino-N-oxide), Zwittergente 3-10, Xwittergente 3-14, Xwittergente SB 3-16, Empigen, NDSB-20, T4G32, E. coli SSB, RecA, 7-deazaG, dUTP, UNG, anionic detergents, cationic detergents, non-ionic detergents, zwittergente, sterols, cations, and any other chemicals, proteins, or cofactors that can alter the amplification efficiency.

As used herein, the term "thermostable", when applied to the enzyme, refers to an enzyme that retains its biological activity at elevated temperatures (for example, at 55°C or more), or retains its biological activity after repeated cycles of heating and cooling. Thermostable nucleotide polymerases are particularly preferred for the present invention, as they eliminate the need to add enzyme before each PCR cycle.

The "polymerase activity" refers to an enzymatic activity that catalyzes the polymerization of deoxyribonucleotides. Generally, the enzyme will initiate synthesis at the 3' end of the primer annular to the target sequence, and will proceed towards the 5' end of the template strand. In certain embodiments, this enzyme is a thermostable DNA polymerase.

Non-limiting examples of thermostable DNA polymerases include, but are not limited to, polymerases isolated from *Thermus aquaticus* (Taq polymerase), *Thermus thermophilus* (Tth polymerase), *Thermococcus coastalis* (Tli or VENT™ polymerase), *Pyrococcus furiosus* (Pfu or DEEPVENT™ polymers), *Pyrococcus woosii* (Pwo polymerase) and other species of *Pyrococcus, Bacillus stearothermophilus* (Bst polymerase), *Sulfolobus acidocaldarius* (Sac polymerase), *Thermoplasma acidophilum* (Tac polymerase), *Thermus rubber* (Tru polymerase) *(Thermus brim)* (Thermus rubber) Tne polymerase), *Thermotoga maritime* (Tma) and other species of *Thermotoga genus* (Tsp polymerase), and *Methanobacterium thermoautotrophicum* (Mth polymerase).

The PCR reaction may contain more than one thermostable polymerase enzyme with complementary properties, resulting in more efficient amplification of the target sequences. For example, a polymerase with a high ability to amplify large segments of nucleotides can be complemented with another polymerase capable of correcting errors that occur during the elongation of the target nucleic acid sequence, thus creating a PCR reaction that can amplify a long target sequence with high Fidelity. The thermostable polymerase can be used in its wild form, or alternatively, the polymerase can be modified to contain a fragment of an enzyme or to contain a mutation that provides beneficial properties to facilitate the PCR reaction. In one embodiment, the polymerase can be Taq polymerase. Many variants of Taq polymerase with improved properties are known and include, but are not limited to, AmpliTaq™, Stoffel fragment, SuperTaq™, SuperTaq™ plus, LA Taq™, LApro Taq™, and EX Taq™.

As already mentioned above, the term "hybridization conditions" refers to conditions that allow the primer or probe to annular to the nucleotide sequence of interest. These conditions are dependent on the temperature and ionic strength of the solution in which the hybridization takes place. These are the stringent conditions. As understood by an experienced technician, annealing stringency can be altered in order to identify or detect identical or related polynucleotide sequences. As will be appreciated by an experienced technician, the *melting* temperature, Tm, can be calculated by formulas known in the art, depending on several parameters, such as the length of the primer or probe in number of nucleotides, or ingredients present in the buffer and conditions. For this, see, for example, T. Maniatis et al., Molecular Cloning: A Laboratory Manual, Cold Spring Harbor Laboratory, Cold Spring Harbor, N.Y., 1982 and J. Sambrook et al., Molecular Cloning: A Laboratory Manual, Cold Spring Harbor Laboratory, Cold Spring Harbor, N.Y., 1989).

The annealing temperature ranges can vary from around 50°C and 65°C, but the primers can be designed to be optimal at around 58°C to 62°C. An additional consideration when designing primers is the content of guanine and cytosine. Generally, the GC content for an initiator can be around 30-70%, but it can be less and can be adjusted appropriately by an experienced technician. The annealing of oligonucleotides complementary or partially complementary to a given target can be obtained by modifying the annealing conditions in order to increase or decrease the stringency, for example, by adjusting the temperature or the salt concentration in the buffer. Such modifications to maintain specificity for *N. meningitidis, H. influenzae* or *S. pneumoniae* can be performed routinely by an experienced technician.

For amplification, a pair of primers of a specific type can be used alone (for example, a *forward* primer and a reverse primer for *N. meningitidis;* a *forward* primer and a reverse primer for *H*. *influenzae;* or a *forward* primer and a reverse primer of *S. pneumoniae,* and so on). *Multiplex* amplification can be used to amplify regions of the target genes of *N. meningitidis, H*. *influenzae* or *S. pneumoniae.* The final concentrations of the primers can be adjusted appropriately, ranging from about 10 pmol to 50 pmol (in 20 µl) of each of the primers represented by SEQ ID Nos: 1 to 6.

The final concentrations of the probes can also be adjusted appropriately by an experienced technician, ranging from about 50 nM to 1000 nM. More preferably, the final concentration ranges from about 100 to about 300 nM, more preferably, from 150 to 250 nM for each of the probes represented by SEQ ID Nos: 7 to 9.

In another aspect of the invention, a method is provided for detecting the presence of *N. meningitidis, H*. *influenzae* or *S. pneumoniae,* simultaneously, from nucleic acids extracted from a biological sample. The method includes mixing the dNTPs, the DNA polymerase, buffer, at least one primer and at least one probe as described in this application, the nucleic acid extracted from the biological sample in a suitable container, and subjecting the container containing the mixture to incubation in a thermal cycler.

In another aspect, the invention provides a method for detecting the presence of *N. meningitidis, H*. *influenzae* or *S. pneumoniae,* comprising carrying out a polymerase chain reaction using at least one or a set of primers selected from the group of *forward* primers of SEQ ID Nos: 1, 3 and 5, and at least one or a set of primers selected from the group of reverse primers of SEQ ID Nos: 2, 4 and 6. The experienced technician is aware of the PCR reaction conditions, in particular, the thermal cycling conditions, for example, temperatures, duration, number of cycles, heating/cooling rate, etc. In a preferred embodiment, the PCR reaction conditions include conditions suitable for a *multiplex* PCR. In another preferred embodiment, said conditions include those suitable for real-time quantitative *multiplex* PCR. In another optional embodiment, said method comprises the step of placing the sample in the presence of probes in conditions suitable for annealing such probes to the amplicon.

In another preferred embodiment, the method comprises the step of detecting at least one amplicon in real time, allowing the assessment of the presence or absence of *N. meningitidis, H*. *influenzae* or *S. pneumoniae* in the sample. This is achieved, without limitation, by the fluorescence intensity or TM measurements of the amplicons.

Fluorescence and TM measurement procedures are known in the art.

In a preferred embodiment, at least one step, preferably several steps, most preferably most steps, are performed on a PCR plate, including those with 24 wells, 48 wells, 96 wells and 384 wells. The use of plates ensures, advantageously, that the samples can be processed in parallel during the reaction. In addition, it allows the method to be performed on a large scale, which saves time.

In another preferred form, at least one step, preferably several steps, more preferably most of the steps are carried out in a thermal cycler. The following are some thermal cyclers for real-time PCR with HRM system: RotorGene Q 5-plex HRM from Qiagen; 7500 Fast Real Time PCR System and Quant Studio 6 by Thermo Fisher, BIO-MA-6000 by INNOVA MOLARRAY; PCRmax Eco 48 from COLE-PARMER among others.

The present invention is illustrated by the examples below, which are intended only to exemplify one of the countless ways of carrying out the invention, however, without limiting its scope. The various modifications or suggestions in the light of them that may be suggested by an experienced technician are included in the spirit and scope of the claims. In particular, although they are suitable for detection via *multiplex* and/or real-time protocols, the methods, oligonucleotides, sets of oligonucleotides and *kits* of the present invention are, of course, also suitable for qualitative *singleplex, duplex, triplex* and similar protocols, Conventional PCR and real-time PCR with Taqman system, and combinations thereof.

### EXAMPLES

### Example 1. Reference microorganisms

The strains used as reference are part of the Collection of Reference Microorganisms in Health Surveillance - CMRVS of the National Institute for Quality Control in Health - INCQS of FIOCRUZ. Reference strains from different serogroups and serotypes were used to guarantee the specificity of the system in detecting the three species studied. Other species were used as negative controls. Table 1 shows the complete list of reference microorganisms used.

**Table 1. Complete list of Reference Microorganisms used**

| **Microorganism** | **Origin** |
|---|---|
| *Haemophilus influenzae* NT | ATCC 49247 |
| *Haemophilus influenzae* type a | ATCC 9006 |
| *Haemophilus influenzae* type b | ATCC 33533 |
| *Haemophilus influenzae* type b | ATCC 10211 |
| *Haemophilus influenzae* type b | ATCC 9795 |
| *Haemophilus influenzae* type c | ATCC 9007 |
| *Haemophilus influenzae* type d | ATCC 9008 |
| *Haemophilus influenzae* type e | ATCC 8142 |
| *Haemophilus influenzae* type f | ATCC 9833 |
| *Neisseria meningitidis* group A | ATCC 13077 |
| *Neisseria meningitidis* group B | ATCC 13090 |
| *Neisseria meningitidis* group C | ATCC 13102 |
| *Neisseria meningitidis* group D | ATCC 13113 |
| *Neisseria meningitidis* group W-135 | ATCC 35559 |
| *Streptococcus pneumoniae* serotype 3 | ATCC 6303 |
| *Streptococcus pneumoniae* serotype 2 | ATCC 6314 |
| *Streptococcus pneumoniae* serotype 33 | ATCC 8333 |
| *Streptococcus pneumoniae* serotype 41 | ATCC 10341 |
| *Streptococcus pneumoniae* serotype 61 | ATCC 10361 |
| *Streptococcus pneumoniae* serotype 19F | ATCC 49619 |
| *Streptococcus pneumoniae* serotype 6B | ATCC 700670 |
| *Streptococcus pneumoniae* serotype 9V | ATCC 700671 |
| *Streptococcus pneumoniae* serotype 14 | ATCC 700672 |
| *Streptococcus pneumoniae* serotype 19A | ATCC 700673 |
| *Streptococcus pneumoniae* serotype 4 | ATCC BAA-334 |
| *Streptococcus pneumoniae* serotype 6A | ATCC BAA-659 |
| *Streptococcus pneumoniae* serotype 51 | ATCC 10351 |
| *Streptococcus pneumoniae* serotype 5 | ATCC BAA-341 |
| *Neisseria perflava* | ATCC 11076 |
| *Moraxella catarrhalis* | ATCC 25238 |
| *Streptococcus agalactiae* | ATCC 13813 |
| *Streptococcus pyogenes* | ATCC 19615 |
| *Klebsiella pneumoniae* | ATCC 13883 |
| *Listeria monocytogenes* | ATCC 15313 |
| *Acinetobactersp.* | ATCC 14293 |
| *Escherichia coli* | ATCC 11775 |

### Example 2. Design of primers and probes

The specific oligonucleotides for each species for use in the Taqman and HRM system of real-time PCR, were designed from the sequences of the target genes unique to each microorganism with the aid of the Oligo Architect SIGMA software (http://www.oligoarchitect.com/). The strings were obtained from GenBank (http://www.ncbi.nlm.nih.gov/genbank/). Table 2 shows the primers and probes used for the PCR amplification reactions with the respective markings on the fluorophores (reporter) and *quencher* probes. They were synthesized at the Carlos Chagas Institute - FOCOCRUZ, Curitiba, Brazil. Each probe was marked with a different fluorophore so that they could be used simultaneously in a multiplex system. The appropriate *quencher* was used for each fluorophore.

**Table 2. Primers and Probes used in the present invention**

| TARGET GENE | PRIMERS | PROBE | MICROORGANISM |
|---|---|---|---|
| *nspA* | F - CAAGCTCTTTAGGTTCTG | [HEX]ATCTCCGCAGGCTACCGCAT[BHQ-1] | *N. meningitidis* |
| | R-GCTGTAAAGTTTGAAATCG | | |
| ***P6*** | F - GAAGGTAATACTGATGAACG | [Cy5]CACCAGAATACAACATCGCATTAGGAC[BHQ-3] | *H. influenzae* |
| | R - TCACCGTAAGATACTGTG | | |
| ***ply*** | F - CCAAGTCTATCTCAAGTTG | [FAM]AGCAGCCTCTACTTCATCACTCTTAC[BHQ-1] | *S. pneumoniae* |
| | R-CTACCTTGACTCCTTTTATC | | |

According to the present invention, *forward* primers are represented by SEQ ID NOs: 1, 3 and 5, respectively, while reverse primers are represented by SEQ ID NOs: 2, 4 and 6, respectively.

The probes used in the optional embodiment of the present invention are represented by SEQ ID NOs: 7, 8 and 9, respectively.

The best concentration of primers and probes for each target was determined to optimize the reaction for both *DNA* extracted from control bacterial strains and for clinical samples (CSF, blood, serum).

### Example 4. Detection limits (LD) of reference bacteria by the Taqman System

All reagents (MasterMix), primers and probes used in the assays described here for the Taqman system were supplied by the Molecular Biology Institute of Paraná (IBMP-PR). In another aspect, any commercial Mastermix for the Taqman system available can be used.

For the determination of the DNA detection limit of the microorganisms used as control, after extraction and determination of the DNA concentration, it was serially diluted and the respective LD and *Cycle Threshold* (CT) for each microorganism were established (Table 3).

CTs are values generated by drawing the *threshold* line. These values indicate in which run cycle the amplification started. All samples above the *threshold* line are considered positive. CTs are tabulated, according to DNA concentrations. Table 3 shows the CT results for Real-Time PCR performed with the uniplex *Taqman* system, to reach the detection limit. A specific LD and CT was determined for each target.

**Table 3. LD by the Taqman uniplex system with dilutions of the genomic DNA of each target. The values in bold indicate the value of LD and its respective CT.**

| **Microorganisms/target** | **DNA concentration** | CT |
|---|---|---|
| | 24.2 ng | 14.15 |
| | | 14.91 |
| | | 14.59 |
| *N. meningitidis*/*nspA* | 242 pg | 18.19 |
| | | 17.43 |
| | | 18.77 |
| | | 28.26 |
| | 2.42 pg | 29.83 |
| | | 29.42 |
| | **242 fg** | **34.79** |
| | | **34.14** |
| | | **34.69** |
| | | 17.91 |
| | 10.9 ng | 17.25 |
| | | 16.43 |
| *H. influenzae*/*P6* | | 21.92 |
| | 109 pg | 21.90 |
| | | 21.56 |
| | **109 fg** | **36.58** |
| | | **35.44** |
| | | **38.21** |
| | | 20.70 |
| | 0.47ng | 20.68 |
| | | 19.68 |
| *S. pneumoniae*/*ply* | | 26.45 |
| | 4.79 pg | 24.69 |
| | | 24.89 |
| | 479 fg | 30.61 |
| | | 30.51 |
| | | 31.20 |
| | **200fg** | **32.76** |
| | | **32.65** |
| | | **32.66** |

Images 1, 2 and 3 show the Quantification Analysis graphs extracted from the run to the LD of all DNA with the detected concentrations and their respective CT, as listed in table 4.

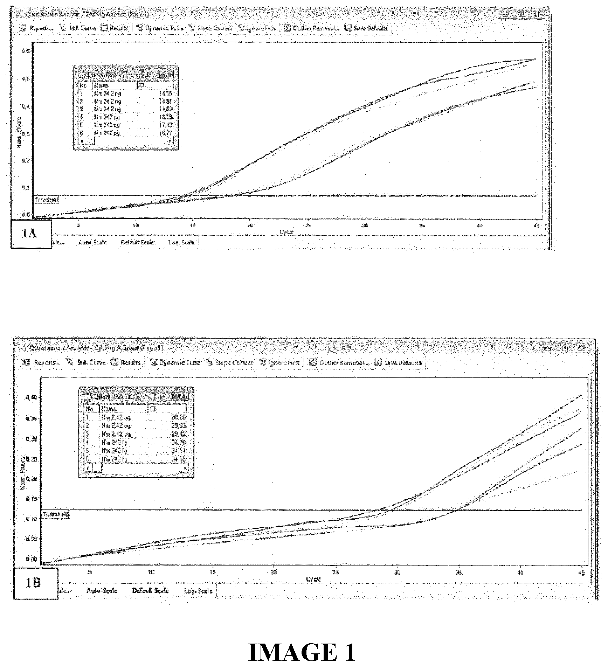

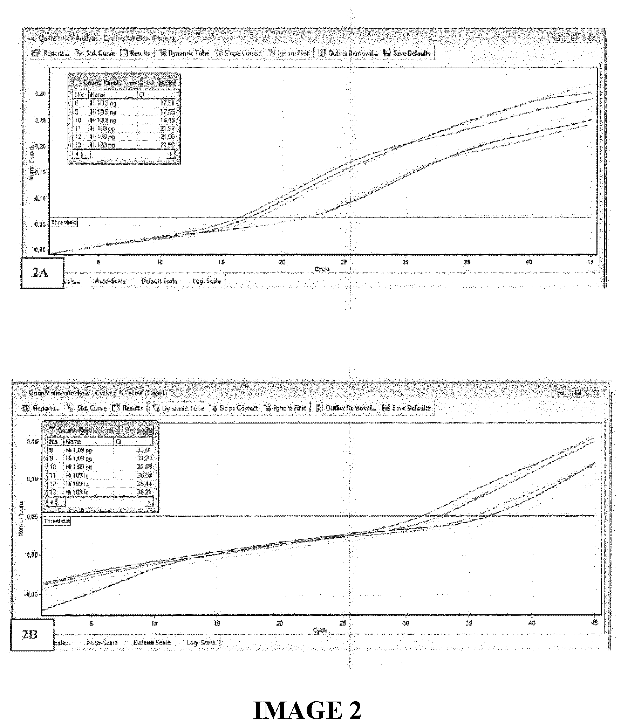

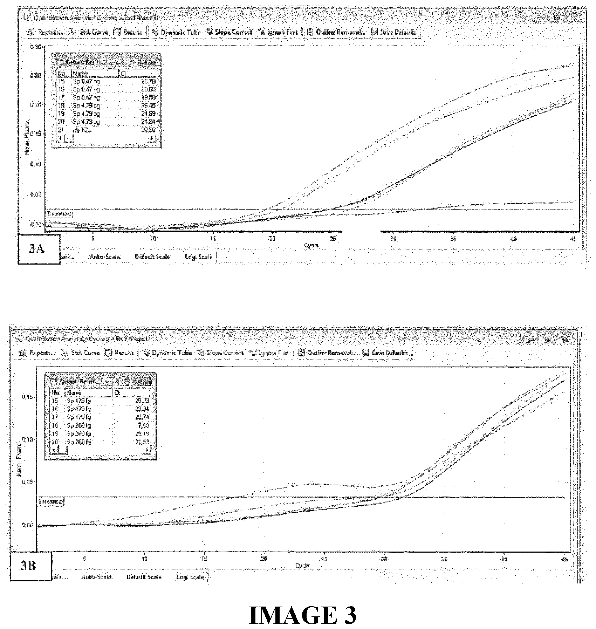

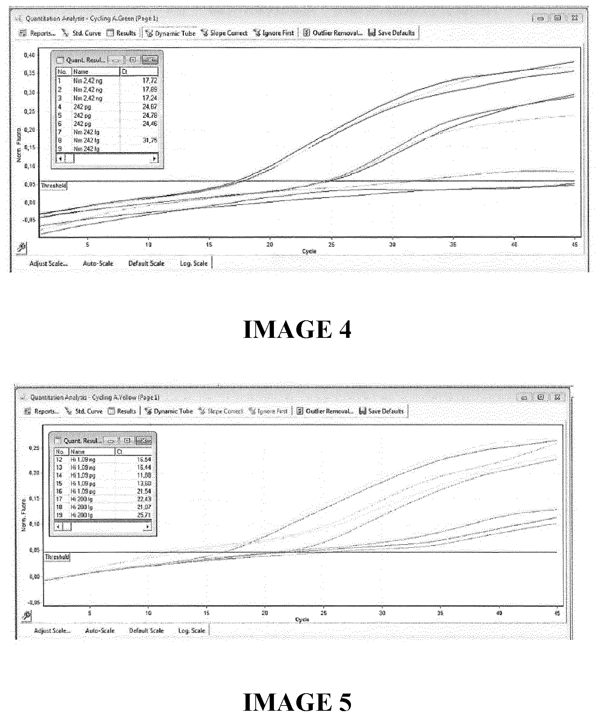

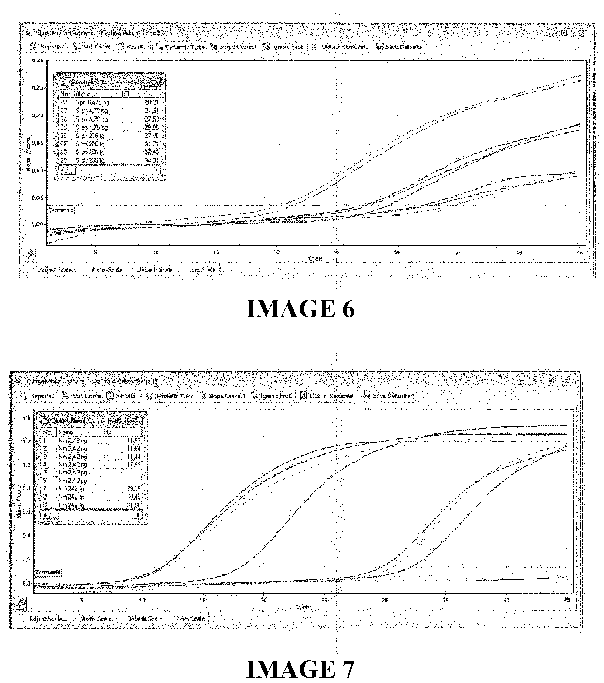

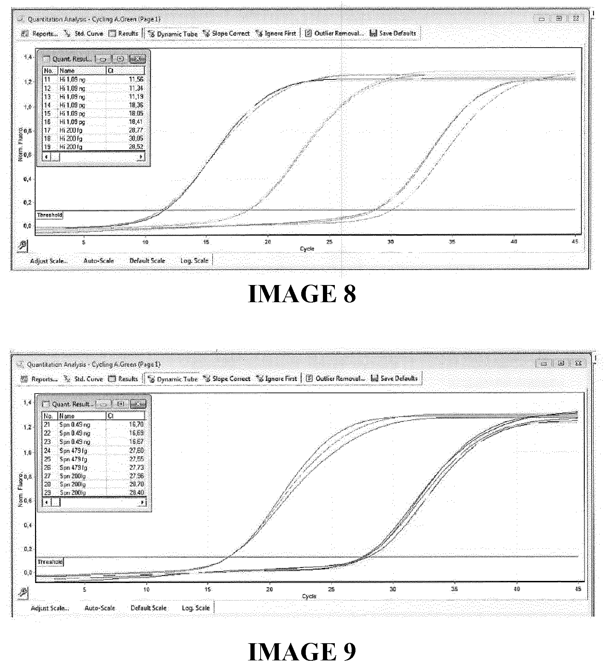

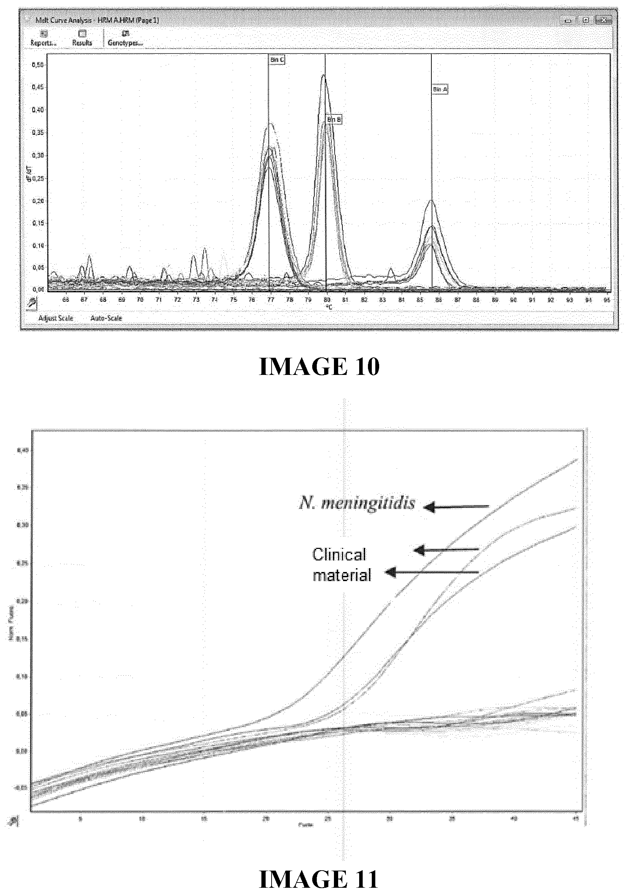

To determine the sensitivity of detection of targets simultaneously (multiplex) by the Taqman system, the same primers and probes were used in a single experiment. The strategy used was the inclusion of the three primer/probe systems and one DNA from each target at a time in a single run since there is no record until the moment of co--infection by more than one of the etiological agents analyzed here. These graphs correspond to figures 4, 5 and 6. The use of the multiplex system saves reagents and time. Table 4 shows in bold the target detection limit by the multiplex.

**Table 4. LD by the Taqman multiplex system with dilutions of the genomic DNA of each target. The values in bold indicate the LD value and its respective CT.**

| **Microorganisms/target** | **DNA concentration (/pL)** | **CT** |
|---|---|---|
| | 2.42 ng | 17.72 |
| | | 17.89 |
| | | 17.24 |
| *N. meningitidis*/*nsp* | 2.42 pg | 24.87 |
| | | 24.78 |
| | | 24.46 |
| | **200 fg** | **31.75** |
| | 10.9 ng | 16.54 |
| | | 16.44 |
| | 10.9 pg | 11.88 |
| *H. influenza*/*P6* | | 13.60 |
| | **200 fg** | **21.54** |
| | | **22.43** |
| | | **21.07** |
| | 0.479 ng | 21.42 |
| | | 20.31 |
| | | 21.31 |
| *S. pneumonia*/*ply* | | 27.53 |
| | 4.7 pg | 29,05 |
| | | 27.00 |
| | **200 fg** | **31.71** |
| | | **32.49** |
| | | **34.31** |

### Example 5. Detection limits uniplex and multiplex by PCR by the HRM system

For the HRM detection system, Qiagen MasterMix "Type-It HRM" Cat was used. 206542 which has the following formula:
- HotStarTaq® PlusDNA Polymerase
- Type-it HRM PCR Buffer (with EvaGreen® dye)
- Q-Solution®
- dNTP mix (dATP, dCTP, dGTP, dTTP)

The primers were purchased from ThermoFisher.

The detection of targets by the HRM system is listed in table 5. Images 7, 8 and 9 show the quantitative analyzes by HRM with the curves and the CTs for each target. Image 1 shows the standard dissociation or *melting* (TM) curves for each target (uniplex). Note that the detection of each target is performed by the peaks of the dissociation curve of each microorganism with different TM, 77°C for S. *pneumoniae,* 80°C for *H influenzae,* and 85 ,8°C for *N. menigitidis,* which can be used unequivocally for the diagnosis of these agents. Images 11 and 12 show the HRM multiplex system with the same strategy used for the Taqman multiplex system, where the three primer/probe systems and one DNA from each target at a time are used in a single run. The tests carried out by both the HRM uniplex and the multiplex systems, showed the same TM results when the genomic DNA of the reference microorganisms were used as a target.

**Table 5. LD by the Taqman uniplex system with dilutions of the genomic DNA of each target. The values in bold indicate the LD value and its respective CT**

| **Microorganisms/target** | **DNA concentration (/L)** | **CT** |
|---|---|---|
| *N. meningitidis*/*nspA* | 2.42 ng | 11.63 |
| | | 11.84 |
| | | 11.44 |
| | 2.42 pg | 17.99 |
| | | **29.56** |
| | **242 fg** | **30.49** |
| | | **31.98** |
| *H. influenzae*/*P6* | 1.09 ng | 11.56 |
| | | 11.34 |
| | | 11.19 |
| | | 18.36 |
| | | 18.05 |
| | 1.09 pg | 18.41 |
| | | **28.77** |
| | | **30.05** |
| | **200fg** | **28.02** |
| *S. pneumoniae*/*ply* | 0.47ng | 16.70 |
| | | 16.69 |
| | | 16.67 |
| | | 27.60 |
| | | 27.55 |
| | 479 fg | 27.73 |
| | | **27.96** |
| | | **28.78** |
| | **200 fg** | **28.40** |

### Example 6. Testing with clinical material by the Taqman System

After the determination of LD by the Taqman system with genomic DNA of reference microorganisms, individually (uniplex) and simultaneous (multiplex), experiments with clinical material of patients with suspected bacterial meningitis by one of the three agents studied were started. Then the results were compared with conventional PCR.

Human clinical material (blood, serum and CSF) received by LACEN-RJ from public hospitals was collected from patients with suspected invasive disease by one of the three agents listed here. Part of this material is sent to our laboratory (LMR/INCQS) in a collaborative effort to detect the etiological agent in cases of negative diagnosis by conventional laboratory tests at LACEN.

The uniplex and multiplex experiments showed similar results. The first experiment by Taqman multiplex demonstrated good specificity in the reaction. Image 11 shows the multiplex test with the reference DNA of *Neisseria meningitidis* and the clinical material in duplicate, indicating that it refers to the mentioned agent. The other samples were negative because the amplification curve was not obtained and all reacted linearly. In image 12, it is possible to observe the multiplex test, with the graph similar to the previous one. In this test, reference DNA from S. *pneumoniae* and clinical material was used, indicating the presence of the microorganism's DNA.

### Example 7. Testing with clinical material by the HRM System

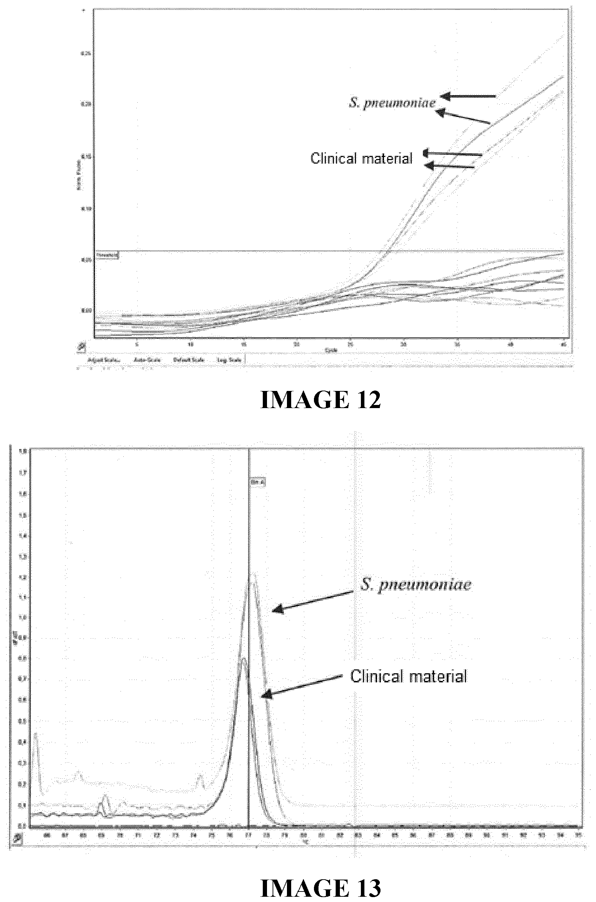

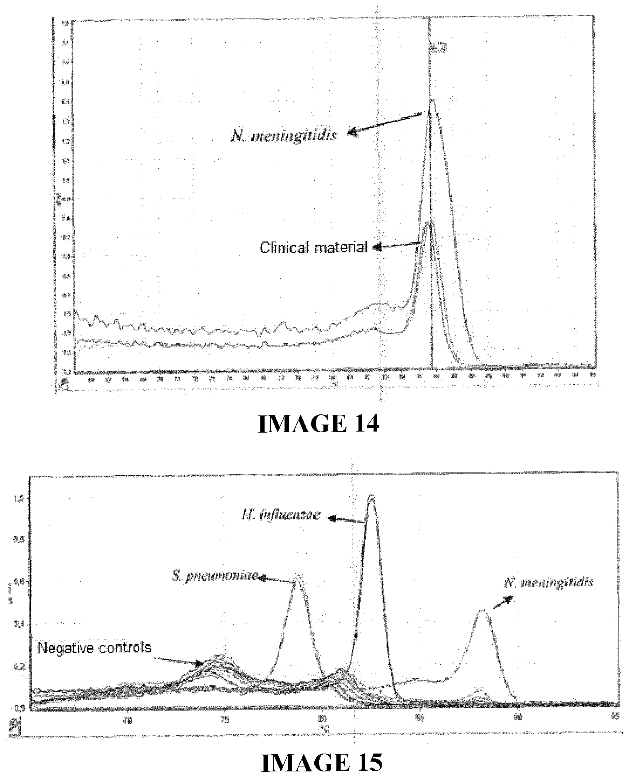

In parallel to the tests carried out with the Taqman system, according to the previous example, experiments with the HRM multiplex system, clinical material for the determination of the etiological agent, were included in the experiments. The detection of the agents was possible due to the differences in *melting* temperature (TM). *N meningitidis* presented TM = 85.8°C, *H. influenzae* = 80°C and S. *pneumoniae* = 77°C. The uni and multiplex tests showed TM at equal temperatures. Figure 13 shows the detection of S. *pneumoniae* and figure 14 shows the detection of *N. meningitidis.*

### Example 8. Comparison between the different PCR systems

The results obtained by the Taqman and HRM systems showed that both methodologies are efficient in the diagnosis of bacterial meningitis, as they presented similar results among them with greater sensitivity for the HRM system as shown in table 6. When compared to conventional PCR, methods based on real-time PCR (Taqman and HRM) showed greater sensitivity. The HRM system, as it does not use a labeled probe, is more cost-effective for the rapid detection of the three etiologic agents analyzed here. The specificity of the primers and probes for each target microorganism was proven through tests with negative control, as listed in table 1 and shown in Image 15. To read the results, conventional PCR requires an additional step of agarose gel electrophoresis to visualize the DNA bands, which is not necessary in qPCR because the result is plotted in graphs and can be viewed in real time, with specific computer software coupled to the thermal cycler. Table 6 compares the DNA detection of *N. meningitidis, H. influenzae* and S. *pneumoniae* from different clinical samples by the Taqman, HRM and conventional PCR systems, all in the simultaneous (multiplex) form. For the Taqman and HRM PCR systems, the same primers (Taqman, HRM) and probes (Taqman) were used. For conventional PCR different primers were used but with the same genes as targets, directed to regions that comprise the same regions amplified by the primers used in the Taqman and HRM systems.

**Table 6. Comparison of detection of etiologic agents by conventional PCR and real-time PCR (Taqman and HRM). (Nm = N meningitidis, Hi = H. Influenzae and Sp = S. Pneumoniae, NEG = negative result).**

| **Clinical Material (n°)** | **Conventional PCR** | **Real-time PCR** | |
|---|---|---|---|
| | | **Taqman** | **HRM** |
| 1473 | Nm | Nm | Nm |
| 022 | Nra | Nm | Nm |
| 505 | Sp | **NEG** | Sp |
| 080 | Sp | Sp | Sp |
| **094** | NEG | NEG | **Nm** |
| 2049 | NEG | NEG | NEG |
| **107** | NEG | NEG | **Sp** |

The sensitivity of the test was assessed by determining the Limits of Detection (LD) shown above. The two detection systems, Taqman and HRM, were also tested against a panel of clinical samples pre-analyzed by conventional PCR with positive results for Nm, Sp and negative. The results are shown in Table 6 and show that in general the Taqman and HRM systems were more sensitive than conventional PCR confirming the presence of DNA from the etiologic agent of positive samples by conventional PCR and some negative ones. All clinical samples used in this panel were obtained from patients with clinical suspicion of invasive disease by one of the etiologic agents, therefore, the detection of bacterial DNA by the Taqman and HRM systems of samples considered negative by conventional PCR shows the greater sensitivity of the two new diagnostic systems. In only one panel sample, the Taqman system did not detect DNA from the etiologic agent that was detected by conventional PCR and HRM. The specificity of the Taqman and HRM systems was evaluated against a panel of reference bacterial strains characterized as invasive already detected in patients with symptoms similar to those caused by the three etiologic agents studied (Table 1). The two systems identified only strains of the species studied here *(N. meningitidis, H. influenzae* and *S. pneumoniae).*

It is evident that the examples above were presented for illustrative purposes only, and that modifications and variations thereof, obvious to those skilled in the art, are considered to be included in the scope of the present invention, as defined in the following claims.

## Claims

1. Oligonucleotide, **characterized by** the fact that it is able to bind to a region of the *Neisseria meningitidis nspA* gene, and is suitable as a primer, said oligonucleotide comprising at least 10 to 15 consecutive nucleotides of the selected sequences of SEQ ID NOs: 1 and 2.

2. Oligonucleotide, **characterized by** the fact that it is able to bind to a region of the *Haemophilus influenzae P6* gene, and is suitable as a primer, said oligonucleotide comprising at least 10 to 15 consecutive nucleotides from the selected sequences of SEQ ID Nos: 3 and 4.

3. Oligonucleotide, **characterized by** the fact that it is able to bind to a region of the *Streptococcus pneumoniae ply* gene, and is suitable as a primer, said oligonucleotide comprising at least 10 to 15 consecutive nucleotides from the selected sequences of SEQ ID Nos: 5 and 6.

4. Oligonucleotide, **characterized by** the fact that it is able to bind to the *nspA* gene segment of *Neisseria meningitidis,* and is suitable as a probe, said oligonucleotide probe comprising at least 8 to 15 consecutive nucleotides of the selected sequence of SEQ ID NO: 7.

5. Oligonucleotide, **characterized by** the fact that it is able to bind to the *P6* gene segment of *Haemophilus influenzae,* and is suitable as a probe, said oligonucleotide probe comprising at least 8 to 15 consecutive nucleotides of the selected sequence of SEQ ID NO: 8.

6. Oligonucleotide, **characterized by** the fact that it is able to bind to the *ply* gene segment of the *Streptococcus pneumoniae,* and is suitable as a probe, said probe oligonucleotide comprising at least 8 to 15 consecutive nucleotides of the selected sequence of SEQ ID NO: 9.

7. Oligonucleotide according to claim 4, 5 or 6, **characterized by** the fact that it is marked with a detectable mark, preferably a fluorescent cluster.

8. Oligonucleotide according to claim 7, **characterized in that** the fluorescent cluster comprises a donor fluorophore pair and a *quencher.*

9. Oligonucleotide set, **characterized by** the fact that it comprises at least two oligonucleotides selected from sequences comprising SEQ ID NOs: 1-6.

10. An oligonucleotide set according to claim 9, **characterized in that** it additionally comprises at least one oligonucleotide selected from sequences comprising SEQ ID NOs: 7-9.

11. Method for simultaneous detection of *Neisseria meningitidis, Streptococcus pneumoniae* and *Haemophilus influenzae,* **characterized by** the fact that it comprises the steps of:
a) producing at least one amplicon using at least two oligonucleotides, such oligonucleotides as defined in claims 1 to 3, and
b) detecting the presence of amplicons.

12. Method according to claim 11, **characterized in that** said step of producing at least one amplicon comprises at least one of amplification by *multiplex* PCR, or in real time.

13. Method according to claim 11, **characterized in that** said step of detecting an amplicon comprises detecting the *melting* temperatures (TM) of the amplicons.

14. Method according to claim 13, **characterized in that** the amplicon of *N. meningitidis* has a TM of 85.8°C; the amplicon of *H. influenzae* has a TM of 80°C; and *S. pneumoniae* amplicon has a TM of 77°C.

15. Method according to claim 11, **characterized in that** said step of detecting an amplicon comprises detecting at least one probe oligonucleotide.

16. Method according to claim 15, **characterized in that** the probe nucleotide comprises at least one oligonucleotide selected from sequences comprising SEQ ID NOs: 7-9.

17. Method according to claim 15, **characterized in that** the probe oligonucleotide is attached to a fluorophore and a *quencher.*

18. Method according to any one of claims 11 to 14, **characterized in that** it allows discrimination between infections by *Neisseria meningitidis, Streptococcus pneumoniae* and *Haemophilus influenzae.*

19. Kit for diagnosis and discrimination of infection by *Neisseria meningitidis, Streptococcus pneumoniae* and *Haemophilus influenzae,* **characterized by** the fact that it comprises at least one oligonucleotide as defined in any one of claims 1 to 3, and b) optionally, instructions for use.

20. Kit according to claim 19, **characterized in that** it additionally comprises at least one set of oligonucleotides as defined in any one of claims 4 to 6.

21. Kit according to claim 20, **characterized in that** the oligonucleotides are probe oligonucleotides, and are attached to at least one fluorophore and a *quencher.*

22. Kit according to any one of claims 19 to 20, **characterized in that** it also includes a negative control and/or a positive reaction control.


SEQUENCE LISTING

<110>   OSWALDO CRUZ FOUNDATION

<120>   OLIGONUCLEOTIDES, OLIGONUCLEOTIDE SET, AND, METHOD FOR
        DETECTION OF ETIOLOGICAL AGENTS FROM BACTERIAL MENINGITIS, KIT
        FOR DIAGNOSIS AND DISCRIMINATION OF ETIOLOGICAL AGENTS OF
        BACTERIAL MENINGITIS

<130>   R000532

<160>   9

<170>   PatentIn version 3.5

<210>   1
<211>   18
<212>   DNA
<213>   Artificial Sequence

<400>   1
caagctcttt aggttctg                                                    18

<210>   2
<211>   19
<212>   DNA
<213>   Artificial Sequence

<400>   2
gctgtaaagt ttgaaatcg                                                   19

<210>   3
<211>   20
<212>   DNA
<213>   Sequência Artificial

<400>   3
gaaggtaata ctgatgaacg                                                  20

<210>   4
<211>   18
<212>   DNA
<213>   Artificial Sequence

<400>   4
tcaccgtaag atactgtg                                                    18

<210>   5
<211>   19
<212>   DNA
<213>   Artificial Sequence

<400>   5
ccaagtctat ctcaagttg                                                   19

<210>   6
<211>   20

<212>   DNA
<213>   Artificial Sequence

<400>   6
ctaccttgac tccttttatc                                                   20


<210>   7
<211>   20
<212>   DNA
<213>   Artificial Sequence

<400>   7
atctccgcag gctaccgcat                                                   20


<210>   8
<211>   27
<212>   DNA
<213>   Artificial Sequence

<400>   8
caccagaata caacatcgca ttaggac                                           27


<210>   9
<211>   26
<212>   DNA
<213>   Artificial Sequence

<400>   9
agcagcctct acttcatcac tcttac                                            26